(19) Europäisches Patentamt
European Patent Office
Office européen des brevets

(11) Veröffentlichungsnummer: **0 014 239**
**A2**

# (12) EUROPÄISCHE PATENTANMELDUNG

(21) Anmeldenummer: 79104684.0

(22) Anmeldetag: 24.11.79

(51) Int. Cl.³: C 07 F 9/50
C 08 F 4/70, C 08 F 10/02

(30) Priorität: 20.01.79 DE 2902203

(43) Veröffentlichungstag der Anmeldung: 20.08.80 Patentblatt 80/17

(84) Benannte Vertragsstaaten: BE FR GB IT NL

(71) Anmelder: CHEMISCHE WERKE HÜLS AG
Postfach 1320
D-4370 Marl 1(DE)

(72) Erfinder: Hänssle, Peter, Dr.
Oelmühle 15
D-4358 Haltern(DE)

## (54) Verfahren zur Herstellung von tertiären 2-Carboxiethyl- und Carboximethylphosphinen sowie deren Salze und Verwendung derselben.

(57) Verfahren zur Herstellung von tertiären 2-Carboxiethyl und Carboximethylphosphinen sowie deren Salze der allgemeinen Formeln $P(CH_2-CH_2-COOMe)_3$ I oder $RP(CH_2-COOMe)_2$ II, wobei Me Wasserstoff, Alkalimetall oder $NR'_4$-Rest mit R' gleich Wasserstoff und/oder Alkyl- oder Arylrest und R ein gegebenenfalls substituierter Arylrest mit 6 bis 14 Kohlenstoffatomen sein kann, indem man die entsprechenden Cyanoalkylphosphine zunächst mit einer wäßrig-alkoholischen Alkalimetallhydroxid-Lösung verseift, dann die wäßrige Lösung der Säuresalze mit einem sauren Ionenaustauscher behandelt und die freien Säuren gegebenenfalls mit einem Alkalimetallhydroxid oder Ammoniak bzw. einem Amin neutralisiert.

Die so hergestellten tertiären 2-Carboxiethyl- und Carboximethylphosphine finden Verwendung als Katalysatorbestandteil bei der Oligomerisierung von Ethylen mit Hilfe eines Nickelkatalysators.

EP 0 014 239 A2

Verfahren zur Herstellung von tertiären 2-Carboxiethyl- und Carboximethylphosphinen sowie deren Salze und Verwendung derselben

Aus der Gruppe der tertiären 2-Carboxiethyl- und Carboximethylphosphine und deren Alkalimetallsalze sind bekannt: Tris-(2-carboxiethyl)-phosphin [Journal of General Chemistry of the USSR 46, 275 (1976)[1]] und Bis-(carboximethyl)-phenylphosphin sowie deren Natrium- und Kaliumsalz [Collect. Czechoslov. Chem. Commun. 43, 57 (1978)[2]].

Solche Phosphine werden gebraucht zur Flammfestausrüstung von Polymeren (1) und zur Komplexierung von Metallionen (2). Gemäß (1) wird Tris-(2-carboxiethyl)-phosphin durch Erhitzen des entsprechenden Nitriles mit einem stark sauren Ionenaustauscher auf 90 bis 95 °C, Absaugen des Ionenaustauschers und Eindampfen der wäßrigen Lösung gewonnen. Bis-(carboximethyl)-phenylphosphin wird nach (2) durch Umsetzung des Reformatsky-Reagenzes $BrZnCH_2COOC_2H_5$ mit Phenyldichlorphosphin, Verseifung des entstehenden Esters $C_6H_5P(CH_2COOC_2H_5)_2$ mit Natronlauge und Behandlung des Natriumsalzes mit 10prozentiger Schwefelsäure erhalten. In beiden Fällen wird die Phosphinocarbonsäure nur in geringer Ausbeute erzeugt. Außerdem ist die angegebene Synthese zur Herstellung von Bis-(carboximethyl)-phenylphosphin vielstufig und damit aufwendig.

Den Carboxialkylphosphinen kommt in zunehmendem Maße technische Bedeutung zu. So ist es aus den deutschen Auslegeschriften 19 55 828 und 20 22 184 bekannt, solche Verbindungen als Liganden für Übergangsmetallkatalysatoren bei der Hydroformylierung von Olefinen einzusetzen. Weiterhin ist es aus den deutschen Offenlegungsschriften 20 53 758, 20 54 009, 20 54 083, 21 59 370, 22 34 734, 22 64 088, 23 41 472, 24 45 362 und 26 56 383 bekannt, tertiäre phosphororganische Verbindungen der allgemeinen Formel $R''_2P-(CH_2)_n-COOM$ mit n = 1 oder 2, M = H oder Alkalimetall und R" gleich einwertiger organischer Rest mit 1 bis 20 C-Atomen als Nickelliganden bei der Ethylenoligomerisierung einzusetzen.

Aufgabe der vorliegenden Erfindung ist es zunächst, ein einfaches und wirtschaftliches Verfahren zur Herstellung von tertiären 2-Carboxiethyl- und Carboximethylphosphinen sowie deren Salze der allgemeinen Formeln
$P(CH_2-CH_2-COOMe)_3$ I oder $RP(CH_2-COOMe)_2$ II,
wobei Me Wasserstoff, Alkalimetall oder $NR'_4$-Rest mit R' gleich Wasserstoff und/oder Alkyl- oder Arylrest und R ein gegebenenfalls substituierter Arylrest mit 6 bis 14 Kohlenstoffatomen sein kann, zu entwickeln.
Weiterhin ist es Aufgabe der Erfindung, die so hergestellten Carboxialkylphosphine für die Ethylenoligomerisierung zu verwenden.

Diese Aufgabe wurde einmal dadurch gelöst, daß man die Cyanoalkylphosphine der allgemeinen Formeln
$P(CH_2-CH_2-CN)_3$ III und $RP(CH_2-CN)_2$ IV,
wobei R die oben erwähnte Bedeutung hat, zunächst mit einer wäßrig-alkoholischen Alkalimetallhydroxid-Lösung verseift, dann die wäßrige Lösung der Säuresalze mit einem sauren Ionenaustauscher behandelt und die freien Säuren gegebenenfalls mit einem Alkalimetallhydroxid oder Ammoniak bzw. einem Amin neutralisiert.

Zum anderen wurde die gestellte Aufgabe durch die Verwendung der nach dem erfindungsgemäßen Verfahren hergestellten tertiären 2-Carboxiethyl- und Carboximethylphosphine als Katalysatorbestandteil bei der Oligomerisierung von Ethylen mit Hilfe eines Nickelkatalysators gelöst.

Die als Ausgangsverbindung dienenden Cyanoalkylphosphine werden nach bekannten Verfahren des Standes der Technik hergestellt [Journal of the Chemical Society, London (1952) 4453; Journal of the American Chemical Society 81, 1103 und 4803 (1959); Acta Chemica Scandinavica B 30, 799 (1976)].

Geeignete Alkohole zur Herstellung der wäßrig-alkoholischen Alkalimetallhydroxidlösungen sind die mit Wasser mischbaren und niedrigsiedenden ($< 100\ ^{\circ}C$) Alkohole, vorzugsweise Methanol und Ethanol. Dafür geeignete Alkalimetallhydroxide sind vorzugsweise Natrium- und Kaliumhydroxid.

Als Ionenaustauscher können alle handelsüblichen Kationenaustauscher, die leicht in die Säureform überführbar sind, eingesetzt werden, vorzugsweise stark saure, $SO_3$-Gruppenhaltige Kationenaustauscher [s. Jander-Wendt, Lehrbuch der analyt. und präp. anorg. Chemie, 3. Auflage (1959), Seiten 44 bis 46]. Sie sollen eine Aufnahmekapazität von etwa 2 mval/ml Harz haben. Zur Überführung der Kationenaustauscher in die $H^{\oplus}$-Form (Säureform) dient vorzugsweise 2n-Salzsäure, anschließend wird mit destilliertem Wasser chlorid- und säurefrei gewaschen. Falls die Kationenaustauscher nicht in genügender Reinheit bezogen werden können, ist darauf zu achten, daß die Verunreinigungen bei dieser Prozedur gleichzeitig mit entfernt werden (z.B. $Fe^{3+}$-Ionen).

Im einzelnen stellt sich das erfindungsgemäße Verfahren wie folgt dar:

Das Cyanoalkylphosphin wird in einem Alkohol-Wasser-Gemisch (bevorzugtes Volumenverhältnis von Alkohol : Wasser = 4 : 1) gelöst, mit der äquivalenten Menge Alkalimetallhydroxid versetzt und das Gemisch anschließend bis zur Beendigung der Ammoniak-Entwicklung unter Rückfluß gekocht. Das Reaktionsgemisch wird dann zur Trockne eingeengt. Man löst den Rückstand in destilliertem Wasser und extrahiert zweimal mit z.B. Diethylether, um nicht umgesetztes Cyanoalkylphosphin zu entfernen. Die wäßrige Lösung wird gegebenenfalls filtriert und das Filtrat wieder bis zur Trockne eingedampft. Auf diese Weise erhält man bereits das Alkalimetallsalz der Phosphinocarbonsäure.

Will man nicht das Alkalimetallsalz, sondern die freie Säure synthetisieren, so kann die letzte Eindampfprozedur entfallen. Die gegebenenfalls filtrierte wäßrige Salzlösung wird dann sofort mit mindestens der äquivalenten Menge des stark sauren Kationenaustauschers in der $H^{\oplus}$-Form behandelt. Vorzugsweise läßt man die Salzlösung über eine Säule laufen, wobei die Tropfgeschwindigkeit so eingestellt wird, daß ein einwandfreier Ionenaustausch gewährleistet ist. Das Wasser der Säurelösung wird abgezogen, und zurück bleibt das gewünschte Carboxialkylphosphin.

Dieses kann gegebenenfalls durch eine Reaktion mit einem Alkalimetallhydroxid, Ammoniak oder einem Amin in ein Alkalimetall- oder Ammoniumsalz überführt werden. Geeignete Amine sind z.B. Triethylamin, Dibutylamin, Butylamin, Diphenylamin, Tridecylamin. Besonders bevorzugt sind sekundäre Amine wegen ihrer großen Basenstärke.

Angesichts der bekannten Verfahren zur Herstellung von Carboxialkylphosphinen ist es überraschend, daß sich Tris-(2-carboxiethyl)-phosphin und Carboximethylphosphine der allgemeinen Formel II sowie deren Salze nach dem beschriebenen und beanspruchten Verfahren in reiner Form und mit hoher Ausbeute herstellen lassen.

Folgende Verbindungen können in vorteilhafter Weise nach dem erfindungsgemäßen Verfahren hergestellt werden: Tris-(2-carboxiethyl)-phosphin, Bis-(carboximethyl)-phenylphosphin sowie deren Ammonium- und Alkali-, vorzugsweise Natrium- und Kaliumsalze. Bei den Salzen ist es nicht erforderlich, daß alle Carboxylgruppen eines Moleküls in Salzform vorliegen müssen.

Die nach dem beanspruchten Verfahren hergestellten Phosphinocarbonsäuren sowie deren Salze finden in erster Linie Verwendung als Katalysatorbestandteil bei der Oligomerisierung von Ethylen mit Hilfe eines Nickelkatalysators. Daneben können sie aber auch z.B. zur Flammfestausrüstung von Polymeren und zur Komplexierung von Metallionen bzw. -atomen eingesetzt werden.

Es sind bereits Verfahren zur Ethylenoligomerisierung bekannt, wobei Ethylen in flüssiger Phase mit einem Nickelkomplex behandelt wird. Der Nickelkomplex enthält ein mit einem zweizähnigen Liganden chelatisiertes Nickelatom. Geeignete zweizähnige Liganden enthalten einen tertiären Organophosphoranteil und eine einzige weitere - aktiven Wasserstoff enthaltende - funktionelle Gruppe an einem Kohlenstoffatom, wobei dieses Kohlenstoffatom entweder direkt an den phosphorhaltigen Molekülteil gebunden oder höchstens durch ein Kohlenstoffatom von ihm getrennt ist. Eine bevorzugte funktionelle Gruppe stellt die Carboxyl- oder Alkalicarboxylatgruppe dar (deutsche Offenlegungsschriften 20 53 758, 20 54 009, 20 54 083, 21 59 370, 22 34 734, 22 64 088, 23 41 472, 24 45 362 und 26 56 383).

Verwendet werden o-Dihydrocarbylphosphinobenzoesäuren und deren Alkalimetallsalze, wie z.B. o-Diphenylphosphinobenzoesäure. Andere bevorzugte tertiäre phosphororganische Verbindungen sind Dihydrocarbylphosphinoessigsäuren und -propionsäuren und ihre Alkalimetallsalze. Sie können durch die allgemeine Formel $R_2P-(CH_2)_n-COOM$ mit n = 1 und 2 und M = H oder Alkalimetall dargestellt werden. R steht für

einen einwertigen organischen $C_{1-20}$-Rest, der zusätzlich Heteroatome in Form von funktionellen Gruppen aufweisen kann, die jedoch keine aktiven Wasserstoffatome enthalten dürfen.

Als Nickelkomponente setzt man entweder Verbindungen des nullwertigen Nickels ein, wie z.B. Bis-[cyclooctadien-(1.5)]-nickel (0), oder man geht von einem zweiwertigen Nickelsalz aus, wie z.B. $NiCl_2 \cdot 6H_2O$, das in Gegenwart der erwähnten tertiären Phosphine und in Gegenwart von Ethylen mit einem Borwasserstoffreagenz in polaren organischen Lösemitteln, wie z.B. Ethylenglykol oder 1.4-Butandiol, reduziert wird.

Die mit solchen Katalysatorsystemen des Standes der Technik nach einem ca. dreistündigen diskontinuierlichen Ethylenoligomerisierungsversuch in 1.4-Butandiol bei 70 °C und 50 bar Ethylendruck erhaltenen Olefingemische haben einen infrarot-spektroskopisch bestimmten n-$\alpha$-Olefinanteil von ca. 96 Gewichtsprozent. Das Ausmaß der Isomerisierung beträgt 2 bis 3 Gewichtsprozent, gemessen am gaschromatographisch ermittelten 2-Buten-Gehalt im anfallenden Buten.

Demgegenüber werden z.B. beim Einsatz von Tris-(2-carboxiethyl)-phosphin Olefingemische mit einem n-$\alpha$-Olefinanteil von 99 bis 100 Gewichtsprozent und einem Isomerisierungsgrad von 0,3 Gewichtsprozent erhalten.
Dabei kann der Nickelkatalysator mit den verschiedensten organischen Liganden komplexiert sein, vorausgesetzt, daß er die erfindungsgemäßen Carboxialkylphosphine enthält.

Im allgemeinen ist das Nickelatom komplex und/oder chemisch an den Phosphor enthaltenden chelatbildenden Liganden sowie an eine dafür ausreichende Anzahl von organischen Komplexbildungs-Liganden gebunden, so daß die Koordinierungszahl des Nickelatoms, welche bevorzugt 4 beträgt, abgesättigt ist. Zusätzliche organische Komplexbildungs-Liganden sind beliebige, von den vorgenannten erforderlichen Phosphor und mehrere Carboxyl- bzw. Carboxylat-Gruppen enthaltenden Liganden verschiedene Liganden, die zur Absättigung der Koordinationszahl des Nickelatoms an dieses komplex gebunden sind. Im allgemeinen sind für diesen Zweck organische Liganden, wie Phosphine, Phosphite, Phosphinoalkylene, Arsine, Stibine oder Bismutine geeignet. Als Komplexbildungs-Liganden bevorzugt werden olefinisch ungesättigte Verbindungen mit 2 bis 20 C-Atomen, bis zu 4 olefinisch ungesättigten Bindungen und bis zu 3 carbocyclischen Ringen. Eine besonders bevorzugte Klasse olefinisch ungesättigter Verbindungen sind $C_{2-12}$-Olefine der allgemeinen Formel

$$\begin{matrix} R^1 & & R^2 \\ | & & | \\ HC & = & CH \end{matrix} \quad ,$$

in der $R^1$ und $R^2$ gleich oder verschieden sind und Wasserstoffatome, Alkyl-, Cycloalkyl-, Alkenyl-, Cycloalkenyl-, Aralkyl-, Aryl- oder Alkarylreste mit bis 8 C-Atomen bedeuten, wobei die Reste $R^1$ und $R^2$ auch gemeinsam einen zweiwertigen aliphatischen $C_{2-10}$-Rest bilden und dabei bis 3 zusätzlich olefinisch ungesättigte Bindungen als einzige ungesättigte C-C-Bindungen aufweisen können. Spezielle Beispiele für Olefine der allgemeinen Formel sind Ethylen, Propylen, Buten-2, Penten-1, Hexen-1, Octen-1, Decen-1, Butadien, Isopren, Octatrien-1.3.5, Octatrien-1.3.7, Cyclopenten, Cyclohepten, Cyclopentadien, Cyclohexadien-1.3, Cyclooctadien-1.5 (COD), Cyclooctatrien und Cyclododecatrien.

Spezielle Beispiele von Nickelchelaten sind Diethylen-bis-(carboximethyl)-phenylphosphin-nickel, Butadien-tris-(2-carboxiethyl)-phosphin-nickel, Cyclooctadien-bis-(2-carboxiethyl)-phenylphosphin-nickel und Cyclooctadien-bis-(2-carboxiethyl)-xylylphosphin-nickel.

Die Nickelchelat-Katalysatoren können nach den verschiedensten Methoden hergestellt werden. Gemäß einem bevorzugten Verfahren des Standes der Technik werden sie durch Behandeln einer Olefin-Nickel-Verbindung mit den erfindungsgemäßen Carboxialkylphosphinen sowie deren Salze erhalten. Eine besondere Klasse von Olefin-Nickel-Verbindungen sind solche der allgemeinen Formel

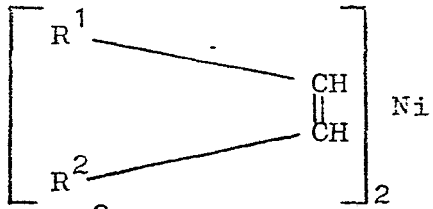

in der $R^1$ und $R^2$ die vorgenannte Bedeutung haben. Spezielle Beispiele sind Bis-(cyclooctadien)-nickel (0), Bis-(cyclooctatetraen)-nickel (0) und Bis-(1.3.7-octatrien)-nickel (0).

Eine weitere Klasse von als Vorprodukte geeigneten Olefin-Nickel-Verbindungen sind π-Allylnickelverbindungen, bei denen ein Nickel enthaltender Anteil an einen π-Allylanteil gebunden ist, welcher durch eine räumliche Verschiebung des vom π-Allylanteil beigetragenen Elektronenanteils zwischen drei miteinander verknüpften C-Atomen charakterisiert ist. Bevorzugte π-Allylanteile weisen 3 bis 12 C-Atome auf und sind ansonsten frei von aliphatischer Ungesättigtheit, außer wenn der π-Allylanteil einen Teil eines geschlossenen Ringsystems darstellt.

Geeignete π-Allylnickelverbindungen sind π-Allylnickelchlorid, π-Allylnickelbromid, π-Crotylnickelchlorid, π-Methylallylnickelchlorid, π-Ethylallylnickeliodid, π-Cyclopentenylnickelbromid, π-Cyclooctenylnickelchlorid, π-Cyclooctadienylnickelchlorid, π-Cinnamylnickelbromid, π-Phenylallylnickelchlorid, π-Cyclohexenylnikkelbromid, π-Cyclododecenylnickelfluorid und π-Cyclododecatrienylnickelchlorid. Andere geeignete π-Allylnickelverbindungen sind π-Allylnickelacetat, π-Methylallylnickeloctoat, π-Allylnickelmethylat, π-Allylnickelethylat und π-Methylallylnickelpropionat.

Weiterhin sind als Vorprodukte Bis-π-allylnickelverbindungen geeignet. Spezielle Beispiele dafür sind Bis-π-allylnickel, Bis-π-methylallylnickel, Bis-π-cinnamyl-

nickel, Bis-π-octadienylnickel, Bis-π-cyclohexenylnickel, π-Allyl-π-methylallylnickel, Bis-π-cyclooctatrienylnickel und Bis-π-crotylnickel.

Die Olefin-Nickel-Katalysatorkomponente und die phosphorhaltigen Ligand-Katalysatorkomponenten werden im allgemeinen in einem Molverhältnis von 0,5 : 1 bis 5 : 1, vorzugsweise 1 : 1, eingesetzt. Der Nickelchelat-Katalysator wird zweckmäßigerweise durch Behandeln der Katalysator-Vorprodukte in einem inerten Verdünnungsmittel vorgeformt, z.B. in solchen Verdünnungsmitteln, welche auch in der anschließenden Oligomerisierung eingesetzt werden können. Nach einer abgewandelten Methode werden die Katalysator-Vorproduktkomponenten zu Beginn des Oligomerisierungsverfahrens in Gegenwart des als Ausgangsmaterial dienenden Ethylens miteinander in Berührung gebracht. Bei jeder der beiden Methoden werden die Katalysator-Vorproduktkomponenten mit Vorteil bei Temperaturen von 25 bis 100 °C kontaktiert.

Neben Nickel(O)-Komplexen kann man auch von zweiwertigen Nickelsalzen ausgehen, die in Gegenwart von Ethylen und den erfindungsgemäßen sekundären oder tertiären Carboxialkylphosphinen sowie deren Salze mit einem Borwasserstoffreagenz reduziert werden. Man benötigt dann keine kostspieligen - oxidativ und thermisch instabilen - Nickelverbindungen, wie z.B. Bis-[cyclooctadien-(1.5)]-nickel (O). Im allgemeinen kann jedes einfache zweiwertige Nickelsalz zur Herstellung des Katalysators verwendet werden, vorausgesetzt, das Nickelsalz löst sich in ausreichendem Maße im Reaktionsmedium. Die Bezeichnung "einfaches zweiwertiges" Nickelsalz bedeutet ein Nickelsalz, in dem das Metall die Oxidationszahl 2 hat und mit Ionen- oder elektrovalenten Bindungen an zwei einwertige anionische Gruppen (z.B. Halogenidgruppen) oder an eine zweiwertige anionische Gruppe (z.B. eine Carbonatgruppe) und nicht komplex oder koordinativ an irgendwelche zusätzlichen Moleküle oder Ionen gebunden ist. Nickelsalze, die Kri-

stallwasser neben einer oder zwei anionischen Gruppen gebunden enthalten, werden jedoch als einfache zweiwertige Nickelsalze bezeichnet. In den meisten Fällen ist ein einfaches zweiwertiges Nickelsalz mit einer Löslichkeit in dem zur Katalysatorherstellung verwendeten Reaktionsverdünnungsmittel oder Lösemittel von mindestens 0,001 Mol pro Liter (0,001 molar) als Ausgangsmaterial für den Nikkelkatalysator geeignet. Ein Nickelsalz mit einer Löslichkeit von mindestens 0,001 Mol/l (0,001 molar) wird vorzugsweise verwendet und insbesondere eines mit einer Löslichkeit von mindestens 0,005 Mol/l (0,005 molar). Sowohl anorganische wie organische zweiwertige Nickelsalze sind als einfache zweiwertige Nickelsalze geeignet. Geeignete anorganische Nickelsalze sind Nickelhalogenide, wie Nickelchlorid, Nickelbromid und Nickeliodid, Nickelcarbonat und Nickelnitrat. Geeignete organische Nickelsalze sind Nickelsalze der Carbonsäuren wie die Nickelsalze der Fettsäuren mit bis zu 10 Kohlenwasserstoffatomen und vorzugsweise mit bis zu 6 Kohlenstoffatomen, z.B. Nickelformiat, Nickelacetat, Nickelpropionat, Nickelhexanoat, Nickeloxalat, Nickelbenzoat und Nickelnaphthenat. Andere geeignete organische Salze sind Nickelbenzolsulfonat, Nickelcitrat, Nickeldimethylglyoxim und Nickelacetylacetonat. Nickelhalogenide, insbesondere Nickelchlorid, und Nickelalkoholate werden unter anderem wegen ihres niedrigen Preises und ihrer Löslichkeit in polaren organischen Lösemitteln vorzugsweise verwendet.

Wie im Falle der nullwertigen Nickelverbindungen wird bei der Katalysatorherstellung im allgemeinen ein Molverhältnis von Nickelsalz zu den phosphorhaltigen Liganden von 0,5 : 1 bis 5 : 1, vorzugsweise 1 : 1, verwendet.

Als Borwasserstoff-Reduktionsmittel können eingesetzt werden: Alkalimetallborwasserstoffe, wie Natriumborwasserstoff, Kaliumborwasserstoff und Lithiumborwasserstoff, Alkalimetallalkoxiborwasserstoffe, bei denen jeder Alkoxirest 1 bis 4 Kohlenstoffatome aufweist, wie Natriumtrimethoxiborwasserstoff und Kaliumtripropoxiborwasserstoff

und Tetraalkylammoniumborwasserstoffe, bei denen jeder Alkylrest 1 bis 4 Kohlenstoffatome aufweist, wie Tetraethylammoniumborwasserstoff. Wegen ihrer Verfügbarkeit im Handel werden vor allem Alkalimetallborwasserstoffe und insbesondere Natriumborwasserstoff verwendet. Bei der Herstellung des Katalysators beträgt das Molverhältnis von Borwasserstoffsalz zu Nickel mindestens 1 : 1. Es scheint keine bestimmte obere Grenze des Verhältnisses von Borwasserstoffreagenz zu Nickel zu bestehen. Aus Gründen der Wirtschaftlichkeit soll das Molverhältnis jedoch einen Wert von 15 : 1 nicht überschreiten. Das bevorzugte Verhältnis von Borwasserstoff zu Nickelsalz liegt gewöhnlich zwischen 1 : 1 und 10 : 1. Die besten Ergebnisse werden mit einem Molverhältnis von etwa 4 : 1 erzielt. Der Katalysator kann zweckmäßigerweise durch Kontaktierung der Ausgangsmaterialien für den Katalysator, d.h. des Nickelsalzes, der phosphororganischen Verbindung und des Borwasserstoff-Reduktionsmittels in Anwesenheit von Ethylen in einem polaren organischen Verdünnungsmittel oder Lösemittel, das durch das Borwasserstoffreagenz nicht reduziert wird, vorzugsweise in dem auch im erfindungsgemäßen Oligomerisierungsverfahren verwendeten polaren organischen Lösemittel, hergestellt werden. In einer bevorzugten Ausführungsform werden das Lösemittel, das Nickelsalz und die Carboxialkylphosphine in Gegenwart von Ethylen vor der Zumischung des Borwasserstoff-Reduktionsmittels kontaktiert. Um den erfindungsgemäßen Katalysator zu erhalten, ist die Anwesenheit von Ethylen während der Katalysatorherstellung wesentlich. Im allgemeinen werden die Ausgangsmaterialien zur Herstellung des Katalysators bei Ethylendrücken von 0,7 bis 100 bar kontaktiert.

Unabhängig von der Wahl der Katalysatorbestandteile wird bei der Katalysatorzubereitung in einem Temperaturbereich von 0 bis 50 °C, vorzugsweise 10 bis 30 °C, gearbeitet. Kontaktierungszeiten von etwa 5 Minuten bis zu 1 Stunde führen im allgemeinen zu befriedigenden Ergebnissen. Bei der Oligomerisierungsreaktion wird Ethylen in Gegenwart eines bei der Reaktionstemperatur flüssigen Lösemittels

mit dem Katalysator kontaktiert. Lösemittelmengen von bis zu etwa 30 l/Mol Ethylen führen zu befriedigenden Ergebnissen. Im allgemeinen wird eine Katalysatorkonzentration (bezogen auf Nickel) in dem Lösemittel von mindestens 0,001 Mol/l und vorzugsweise von 0,005 bis 0,05 Mol/l gewählt.

Als Lösemittel können sowohl für den Einsatz von Nickel(0)-verbindungen als auch von Nickel(II)-salzen nichtpolare organische Lösemittel, wie aliphatische oder aromatische Kohlenwasserstoffe verwendet werden. Vorzugsweise werden jedoch Sauerstoff, Schwefel, Stickstoff und Phosphor in funktionellen Gruppen, wie in Hydroxi-, Alkoxi-, Aryloxi-, Carbalkoxi-, Alkanoyloxi-, Cyan-, Amino-, Alkylamino-, Dialkylamino-, Amid-, N-Alkylamid-, N,N-Dialkylamid- und Sulfonylalkylgruppen enthaltende polare organische Verbindungen als Lösemittel verwendet. Solche organischen Lösemittel sind Glycerintriacetat, Pentaerythrittetraacetat, Diethylenglykoldiacetat, Butylpropionat, Phenylacetat, Dioxan, Tetrahydrofuran, Tetrahydropyran, Dimethoxiethan, Diethylenglykoldimethylether, Dibutylether, Anisol, 1.4-Dimethoxibenzol, p-Methoxitoluol, Methanol, Trifluorethanol, Trifluorpropanol, sek. Butanol, Perfluorbutanol, Octanol, Dodecanol, Cyclopentanol, Cyclohexanol, Glycerin, Trimethylenglykol, Kresol, p-Chlorphenol, m-Bromphenol, 2.6-Dimethylphenol, p-Methoxiphenol, 2.4-Dichlorphenol, Kohlensäureethylen-, -propylen- oder -butylenester, Acetonitril, Propionitril, Butylamin, Dibutylamin, Trihexylamin, N-Methylpyrrolidin, N-Methylpiperidin, Anilin, N.N-Dimethylformamid, N.N-Dimethylacetamid, Tetramethylensulfon (Sulfolan), Dimethylsulfoxid, Trimethylphosphat, Triethylphosphat, Tributylphosphat, Hexamethylphosphorsäuretriamid. Vorzugsweise verwendete Lösemittel sind Alkandiole mit 2 bis 10 Kohlenwasserstoffatomen, wie Ethylenglykol und Propylenglykol. Vorzugsweise werden Alkandiole mit 4 bis 6 Kohlenstoffatomen, wie 1.4-Butandiol und 2.5-Hexandiol, verwendet.

Polare organische Lösemittel werden deshalb vorzugsweise verwendet, weil das Produktgemisch der Ethylenoligomerisierung in ihnen praktisch unlöslich ist. Die Verwendung eines polaren organischen Lösemittels, wie z.B. eines Alkandiols, führt zur Bildung eines zweiphasigen Reaktionsgemisches, d.h. zu einer das Ethylenoligomerisierungsproduktgemisch, nämlich die α-Olefine, enthaltenden Phase und zu einer zweiten, den Nickelkatalysator und das Lösemittel enthaltenden Phase. Bei der Bildung eines zweiphasigen Reaktionsgemisches kann das Ethylenoligomerisierungsprodukt ohne weiteres abgetrennt werden, und die den Katalysator enthaltende Lösemittelphase kann wieder zur Ethylenoligomerisierung verwendet werden. Wie vorstehend bereits dargelegt, werden die polaren organischen Lösemittel auch wegen ihrer Verwendung bei der Herstellung des Katalysators vorzugsweise verwendet. Auch Wasser, das einen bestimmten Anteil eines polaren organischen Lösemittels enthält, kann als Verdünnungs- oder Lösemittel verwendet werden. Gemische aus Wasser und einem polaren organischen Lösemittel bestehen im allgemeinen zu 40 bis 90 Volumenprozent aus diesem Lösemittel und zu 10 bis 60 Volumenprozent aus Wasser.

Es ist unkritisch, nach welcher speziellen Methode die Kontaktierung des Ethylens mit dem Katalysator im Verlauf der Oligomerisierung erfolgt. Gemäß einer Ausführungsform werden die Katalysatorbestandteile und das Lösemittel unter Ethylen oder einem Schutzgas, wie Stickstoff oder Argon, in einen Autoklaven oder einen ähnlichen Druckreaktor eingespeist, bei Verwendung eines Schutzgases wird dieses durch Ethylen verdrängt, und ein bestimmter Ethylendruck wird eingestellt. Das Reaktionsgemisch wird unter Rühren während der gewünschten Reaktionsdauer bei der Reaktionstemperatur und dem entsprechenden Druck gehalten (diskontinuierliches Verfahren). Bei Ausführungsformen, bei denen ein polares organisches Lösemittel verwendet wird und ein zweiphasiges Reaktionsgemisch gebildet wird, kann Ethylen kontinuierlich in eine den Katalysator und das Lösemittel enthaltende Reaktionszone eingeleitet und das gebildete Oligomerengemisch gleichzeitig aus der Reaktionszone abgezogen werden (kont. Verfahren). Unabhängig von der jeweiligen Ausführungsform wird die Oligomerisierung bei folgenden Temperaturen und Drükken durchgeführt: Geeignete Reaktionstemperaturen sind im allgemeinen 10 bis 250 °C, vorzugsweise 20 bis 100 °C. Geeignete Drücke sind 0,7 bis 350 bar Überdruck, vorzugsweise 7 bis 150 bar Überdruck.

Die Oligomerisierungsprodukte werden vom Reaktionsgemisch mittels üblicher Verfahren abgetrennt und gewonnen, wie durch fraktionierte Destillation, selektive Extraktion, Filtration und Adsorption.

Das Reaktionslösemittel, der Katalysator und gegebenenfalls nicht umgesetztes Ethylen können zur weiteren Verwendung wieder in das Reaktionsgefäß zurückgeführt werden. Wird bei der Oligomerisierung ein Katalysator aus Nickel-(II)-salz, erfindungsgemäßem Carboxialkylphosphin und einem Borwasserstoffreagenz in einem polaren organischen Lösemittel verwendet, kann verbrauchter Katalysator, d.h. nicht mehr für die Ethylenoligomerisierung aktiver Katalysator, durch Reaktion mit zusätzlichem Borwasserstoff-Reduktionsmittel und zusätzlichem Nickel(II)-salz in den oben angegebenen Molverhältnissen regeneriert werden. Es ist kein weiterer Zusatz von phosphororganischen Verbindungen zur Regenerierung des verbrauchten Katalysators mehr erforderlich.

Auch die mit Hilfe der erfindungsgemäßen Carboxialkylphosphine erhaltenen Ethylenoligomerisate (im wesentlichen n-$\alpha$-Olefine) liegen hinsichtlich ihrer C-Zahl-Verteilung nach einem geometrischen Verteilungsschema vor. H. Weßlau, Liebigs Ann. 629, 198 (1960), leitete für den katalytischen Ablauf der $\alpha$-Olefin-Synthese folgende Verteilungsfunktion ab:

$$x_p = \frac{ß}{(1 + ß)^p}$$

$x_p$ bedeutet den Molenbruch der Olefine, die im Reaktionsgemisch die Zusammensetzung $CH_2=CH-(C_2H_4)_{p-1}-C_2H_5$ zeigen (p = Zahl der Aufbauschritte aus Ethyleneinheiten, Ethylen (p = 0) wird nicht mitgezählt). Der Parameter ß gibt das Verhältnis der Geschwindigkeiten von Abbruch der Alkylkette am Nickel zu Wachstum (Aufbau) der Kette an, also ß $= \frac{V_a}{V_w}$ . Die Gleichung von H. Weßlau läßt sich in eine Form überführen, die die Verteilung anschaulicher als Gewichtsbruch mp ausdrückt:

$$mp = \frac{ß^2}{1+2\ ß} \cdot \frac{(p+1)}{(1+ß)^p} \qquad (p \geqq 1;\ 100 \cdot mp = Gew.\%)$$

Zur Bestimmung des Parameters ß genügt es, für zwei gaschromatographisch gut erfaßbare n-α-Olefine des Gemisches das Verhältnis der nach folgender Gleichung zu erwartenden relativen Gewichtsmenge (in g oder Gew.%) aufzustellen und daraus ß zu eliminieren (Dissertation R. Streck, RWTH Aachen, 1961):

$$m_{rel} = (p + 1) \ . \ (1 + ß)^{-p}$$

z.B. $$\frac{\%\ Hexen-(1)}{\%\ Octen-(1)} = \frac{3}{4} \cdot \frac{(1 + ß)^{-2}}{(1 + ß)^{-3}} = \frac{3}{4}\ (1 + ß)$$

$$ß = \frac{4 \cdot \%\ Hexen-(1)}{3 \cdot \%\ Octen-(1)} - 1$$

oder $$\frac{\%\ Hexen-(1)}{\%\ Decen-(1)} = \frac{3}{5} \quad \frac{(1 + ß)^{-2}}{(1 + ß)^{-4}} = \frac{3}{5}\ (1 + ß)^2$$

$$ß = \sqrt{\frac{5 \ . \ \%\ Hexen-(1)}{3 \ . \ \%\ Decen-(1)}} - 1$$

Ethylenoligomerisate, besonders diejenigen mit 12 bis 20 Kohlenstoffatomen, sind wertvolle Ausgangsstoffe für Detergentien, wie α-Olefinsulfonate, Alkoholoxethylate und Aminoxide. Die niedrigeren Olefine werden z.B. auf synthetische Schmieröle oder Weichmacheralkohole verarbeitet, die höheren werden u.a. als Wachse eingesetzt.

Die Erfindung wird anhand der nachfolgenden Beispiele und Vergleichsbeispiele erläutert.
Alle Prozentangaben sind, sofern nicht anders erwähnt, Gewichtsprozente.
Die analytischen Kenndaten wurden nach bekannten Methoden (Gaschromatographie, Infrarotspektroskopie und $^{1}H$- und $^{13}C$-Kernresonanzspektroskopie) ermittelt.

Beispiel 1

Zu 116 g (0,6 Mol) Tris-(2-cyanoethyl)-phosphin in einem 500 ml-Dreihalskolben werden 101 g KOH (1,8 Mol) - gelöst in 190 ml Methanol und 50 ml destilliertem Wasser - gegeben. Das Gemisch wird bei einer Ölbadtemperatur von 100 °C ca. 90 Stunden unter Rückfluß gehalten, wobei sich Ammoniak entwickelt, das mit Argon kontinuierlich in eine eingestellte Salzsäure geleitet wird. Das Ende der Reaktion ist am Farbumschlag eines der eingestellten Salzsäure zugesetzten Indikators zu erkennen. Das Reaktionsgemisch wird am Rotationsverdampfer zur Trockne eingeengt. Man löst den Rückstand in 300 ml destilliertem Wasser und schüttelt zweimal mit Diethylether aus. Die wäßrige Phase wird filtriert und das Filtrat zur Trockne eingedampft. Man erhält 207 g, entsprechend 95 % der Theorie $P(CH_2CH_2COOK)_3$ als weißes Pulver.

IR(KBr): breite $\nu_{COO^{\ominus}}$-Bande bei 1550 bis 1600 $cm^{-1}$
$^{1}H$-NMR($D_2O$): Multiplett bei 7 bis 8,4 $\tau$

$^{13}C$-NMR($H_2O$): $P - \underset{a}{CH_2} - \underset{b}{CH_2} - \underset{c}{\overset{O}{\overset{\|}{C}}} - OK$

a: 22,9 ppm $^{1}J_{PC}$ = 52 Hz
25,2 ppm

b: 29,7 ppm $^{2}J_{PC}$ = 2 Hz

c: 180,9 ppm $^{3}J_{PC}$ = 13 Hz
181,5 ppm

Das Kaliumsalz wird in 300 ml destilliertem Wasser gelöst und über eine Säule mit 900 ml stark saurem Kationenaustauscher (LEWATIT S 100®) in der $H^{\oplus}$-Form geleitet. Die Tropfgeschwindigkeit wird auf 2 ml/min eingestellt. Die Umwandlung des Alkalimetallsalzes in die freie Säure ist am Ausgang der Ionenaustauschersäule mittels pH-Papier leicht kontrollierbar. Das Wasser der Säurelösung wird am Rotationsverdampfer abgezogen. Zurück bleibt Tris-(2-carboxiethyl)-phosphin als glasartige gelbe Masse. Die Substanz ist sehr gut in Wasser und Ethylenglykol löslich.

Ausbeute: 142,5 g = 100 % d.Th., bezogen auf das Salz

Schmelzpunkt: 120 °C

IR (KBr): 1720 $cm^{-1}$ $\nu_{COOH}$

$^1H$-NMR ($H_2O$ + 10 % HCOOH): Multiplett bei 6,9 - 8 $\tau$

$^{13}C$-NMR($H_2O$): $P - \underset{a}{CH_2} - \underset{b}{CH_2} - \underset{c}{\overset{O}{\overset{\|}{C}}} - OH$

| | | | |
|---|---|---|---|
| a: | 14,9 ppm<br>17,1 ppm | $^1J_{PC}$ | = 49,5 Hz |
| b: | 30 ppm | $^2J_{PC}$ | $\angle$ 1 Hz |
| c: | 178,1 ppm<br>178,4 ppm | $^3J_{PC}$ | = 6,6 Hz |

<u>Vergleichsbeispiel A</u>

Nach SU-PS 484 221 bzw. Journal of General Chemistry of the USSR <u>46</u>, 275 (1976).

Eine Mischung von 58 g (0,3 Mol) Tris-(2-cyanoethyl)-phosphin, 450 ml stark saurem Ionenaustauscher (LEWATIT S 100®) in der $H^{\oplus}$-Form und 500 ml destilliertem Wasser wird 16 Stunden lang bei 90 bis 95 °C gerührt. Der Ionenaustauscher und unverändertes Tris-(2-cyanoethyl)-phosphin werden von der heißen Lösung abfiltriert und mit 200 ml heißem destilliertem Wasser gewaschen. Aus dem abgekühlten Filtrat scheiden sich weitere 11 g Tris-(2-cyanoethyl)-phosphin ab. Man filtriert ab und schüttelt das Filtrat mit 100 ml Diethylether aus. Die wäßrige Lösung wird eingeengt bis auf 50 ml, wobei erneut 2 g Tris-(2-cyanoethyl)-phosphin ausfallen, die abfiltriert werden.

Anschließend engt man bis zur Trockne ein. Man erhält 4,7 g, entsprechend 6,3 % einer gelben zähen Masse. IR- und NMR-Spektren weisen auf ein unsauberes Tris-(2-carboxiethyl)-phosphin hin.

Beispiel 2

In einem 250 ml-Dreihalskolben werden 18,8 g (0,1 Mol) Bis-(cyanomethyl)-phenylphosphin mit einer Lösung von 8 g NaOH (0,2 Mol) in 20 ml Methanol und 5 ml destilliertem Wasser vermischt. Das Reaktionsgemisch wird ca. 90 Stunden bis zur beendeten Ammoniakentwicklung unter Rückfluß gehalten. Man engt am Rotationsverdampfer zur Trockne ein, löst den Rückstand in 100 ml destilliertem Wasser und ethert zweimal mit je 100 ml aus. Die wäßrige Phase wird vollständig eingedampft. Man erhält 24,3 g, entsprechend 90 % d.Th., $C_6H_5 - P(CH_2COONa)_2$ als weißes Pulver.

IR (KBr): 1585 $cm^{-1}$ ($\nu_{COO^{\ominus}}$)

Das Natriumsalz wird in 100 ml destilliertem Wasser gelöst und über eine Säule mit 120 ml stark saurem Ionenaustauscher (LEWATIT S 100®) in der $H^{\oplus}$-Form geleitet. Man wäscht mit 500 ml destilliertem Wasser nach. Anschließend wird das Wasser am Rotationsverdampfer abgezogen. Zurück bleibt Bis-(carboximethyl)-phenylphosphin als weißes Pulver.

Ausbeute: 20,3 g $\hat{=}$ 100 % d.Th. (bezogen auf das Na-Salz)

Schmelzpunkt: 136 °C

IR (Film): 690 und 750 $cm^{-1}$ (CH-wagging für 5 benachbarte H-Atome), 1440 $cm^{-1}$ (P-Phenyl), breite Bande bei 1710 $cm^{-1}$ ($\nu_{COOH}$)

$^1$H-NMR (d-Methanol): 2,3 $\tau$ (mc, 5 H)
6,95 $\tau$ (s, 4 H)

Vergleichsbeispiel B

Bis-(carboximethyl)-phenylphosphin und sein Natriumsalz werden nach einer bekannten Vorschrift in Collection Czechoslov. Chem. Commun. 43, 57 (1978) dargestellt. Man erhält 6 % einer gelben zähen Masse mit den analytischen Daten wie in Beispiel 2.

Beispiele 3 und 4 sowie Vergleichsbeispiele C bis E

2,5 mMol der in Tabelle 1 angeführten Phosphinliganden werden unter Argon in 500 ml Lösemittel gelöst. Danach suspendiert man darin 2,5mMol (≙ 0,7 g) $Ni(COD)_2$. Diese Lösung bzw. Suspension gibt man unter Argon in einen 5 l-Stahlautoklaven. Man saugt das Schutzgas ab und läßt unter Rühren (1000 U/Min.) ca. 10 bar Ethylen einströmen. Dann wird der Autoklaveninhalt in ca. 5 Minuten auf 70 °C aufgeheizt und der Ethylendruck auf 50 bar erhöht. Bei einem konstanten Druck von 50 bar und einer Rührgeschwindigkeit von 1000 U/Min. oligomerisiert man 165 Minuten. Die Ergebnisse sind in Tabelle 1 zusammengefaßt. Dabei wurde der prozentuale Anteil von 2-Buten im Gesamtbuten gaschromatographisch bestimmt. Der n-α-Olefingehalt wurde IR-spektroskopisch vom Gesamtoligomerisat bestimmt.

Tabelle 1

| Beispiel Nr. | Ligand | Lösemittel | Katalysatoraktivität (g Olefine/g Ni·h) | Isomerisierung (% 2-Buten im Buten) | ß | n-α-Olefingehalt (%) |
|---|---|---|---|---|---|---|
| 3 | $P(CH_2CH_2COOH)_3$ | 50 ml Glykol +450 ml 1.4-Butandiol | 750 | 0,3 | 2,2 | 99 - 100 |
| 4 | $C_6H_5P(CH_2COONa)_2$ | 500 ml 1,4-Butandiol | 100 | - | Wachs | - |
| Vergl.-Beisp. Nr. | | | | | | |
| C | $(C_6H_5)_2P\ CH_2COONa$ | " | 820 | 1,8 | 1,6 | 96 |
| D | $(C_6H_5)_2PCH_2COOH$ | " | 1570 | 2,5 | 0,7 | 96 |
| E | $(C_6H_5)_2PCH_2CH_2COOH$ | " | 270 | 3,0 | 1,1 | 96 |

0014239

Tabelle 3

| Beisp. Nr. | Temp. (°C) | Druck (bar) | Olefinausbeute (g) | Isomerisierung (% 2-Buten in Buten) | ß | IR-Doppelbindungsverteilung (%) | | |
|---|---|---|---|---|---|---|---|---|
| | | | | | | n-α- | iso- | tr.-Olef. |
| 14 | 60 | 20 | 158 | 1,9 | 1,5 | 61 | 28 | 11 |
| 15 | 60 | 50 | 240 | 0,7 | 0,4 | 99 | 0 | 1 |

Beispiel 16

6,6 mMol des Phosphinalkylens $(C_6H_5)_3P = CH - COOC_2H_5$ und 13,3 mMol Tris-(2-carboxiethyl)-phosphin werden unter Argon in 500 ml Ethylenglykol gelöst. Nach Suspension von 20 mMol $Ni(COD)_2$ oligomerisiert man bei 60 °C und 50 bar Ethylendruck.

Olefinausbeute: 335 (g)

Analytische Daten:

| Isomerisierung (% 2-Buten in Buten) | ß | IR-Doppelbindungsverteilung (%) | | |
|---|---|---|---|---|
| | | n-α-Olef. | iso-Olef. | tr.-Olef. |
| 0,7 | 0,3 | 93 | 0 | 7 |

Beispiele 17 bis 21

20 mMol Tris-(2-carboxiethyl)-phosphin werden unter Argon in 500 ml Ethylenglykol gelöst. Anschließend gibt man wechselnde Mengen Natriumhydroxid hinzu und suspendiert dann in dieser Lösung 20 mMol $Ni(COD)_2$. Man oligomerisiert bei 60 °C und 20 bar Ethylendruck 165 Minuten lang. Ergebnisse siehe Tabelle 4.

Beispiele 5 bis 13

Man löst unter Argon 2,5 mMol Tris-(2-carboxiethyl)-phosphin in einem Lösemittelgemisch aus 50 ml Ethylenglykol und 450 ml eines in Tabelle 2 angegebenen Lösemittels. Danach suspendiert man darin 2,5 mMol $Ni(COD)_2$ und oligomerisiert 165 Minuten lang bei 70 °C und 50 bar Ethylendruck. Ergebnisse siehe Tabelle 2.

Tabelle 2

| Beispiel Nr. | Lösemittel (50 ml Glykol und 450 ml Lösemittel) | Katalysatoraktivität (g Olefine/ g Ni·h) | Isomerisierung (% 2-Buten in Buten) | ß | n- α-Olefingehalt (%) |
|---|---|---|---|---|---|
| 5 | Ethylenglykol | 350 | 0,3 | 4 | 99 |
| 6 | 1,2-Propylencarbonat | 60 | 0,9 | $C_4$ | - |
| 7 | 1,3-Butandiol | 520 | 0,4 | 4 | 100 |
| 8 | 1,2-Propylenglykol | 660 | 0,4 | 5 | 93 |
| 9 | Ethylencarbonat | 70 | 0,8 | $C_4$ | - |
| 10 | 1,3-Propylenglykol | 390 | 0,2 | 5 | 100 |
| 11 | 1,4-Butandiol | 750 | 0,3 | 2,2 | 99-100 |
| 12 | 1,5-Pentandiol | 500 | 0,6 | 3,5 | 100 |
| 13 | Dimethylsulfoxid | 25 | 0,9 | $C_4$ | - |

Beispiele 14 und 15

10 mMol Tris-(2-carboxiethyl)-phosphin und 10 mMol Triisopropylphosphin werden unter Argon in 500 ml Ethylenglykol gelöst. Anschließend suspendiert man in dieser Lösung 20 mMol $Ni(COD)_2$ und oligomerisiert bei verschiedenen Drücken und Temperaturen 165 Minuten lang, wie in Tabelle 3 angegeben. Mit $Ni(COD)_2$ und Triisopropylphosphin allein erreicht man keine Oligomerisierung.

Tabelle 4

| Beisp. Nr. | mMol NaOH | Olefinausbeute (g) | Isomerisierung (% 2-Buten in Buten) | β | IR-Doppelbindungsverteilung (%) | | |
|---|---|---|---|---|---|---|---|
| | | | | | n-α- | iso- | tr.-Olefine |
| 17 | 6,7 | 213 | 1,7 | 13 | 59 | 35 | 6 |
| 18 | 10 | 298 | 2,0 | 12 | 56 | 37 | 7 |
| 19 | 20 | 229 | 4,9 | 16 | 45 | 47 | 8 |
| 20 | 40 | 109 | 5,2 | 15 | 49 | 41 | 10 |
| 21 | 60 | 47 | 3,9 | $C_4$ | | | |

Vergleichsbeispiel F

20 mMol des Natriumsalzes der Diphenylphosphinopropionsäure werden unter Argon in 500 ml Ethylenglykol gelöst. Anschließend suspendiert man in dieser Lösung 20 ml $Ni(COD)_2$. Man oligomerisiert bei 60 °C und 20 bar Ethylendruck 165 Minuten lang.

Olefinausbeute: 11 g Buten mit einem Buten-2-Gehalt von 6,5 Gewichtsprozent.

Beispiele 22 bis 25

20 mMol Tris-(2-carboxiethyl)-phosphin werden unter Argon in 500 ml Ethylenglykol gelöst. Anschließend gibt man wechselnde Mengen Dibutylamin hinzu und suspendiert dann in dieser Lösung 20 mMol $Ni(COD)_2$. Man oligomerisiert bei 60 °C und 20 bar Ethylendruck 165 Minuten lang. Ergebnisse siehe Tabelle 5.

Tabelle 5

| Beisp. Nr. | mMol $Bu_2AlH$ | Olefinausbeute (g) | Isomerisierung (% 2-Buten in Buten) | ß | IR-Doppelbindungsverteilung (%) | | |
|---|---|---|---|---|---|---|---|
| | | | | | n-α- | iso- | tr.-Olefine |
| 22 | 6,7 | 283 | 3,0 | 14 | 52 | 40 | 8 |
| 23 | 10 | 299 | 2,9 | 15 | 55 | 37 | 8 |
| 24 | 20 | 269 | 2,4 | 12 | 49 | 43 | 8 |
| 25 | 40 | 84 | 2,0 | 13 | 71 | 22 | 7 |

Patentansprüche

1. Verfahren zur Herstellung von tertiären 2-Carboxiethyl- und Carboximethylphosphinen sowie deren Salze der allgemeinen Formeln

$P(CH_2 - CH_2 - COOMe)_3$ I oder $RP(CH_2 - COOMe)_2$ II,

wobei Me Wasserstoff, Alkalimetall oder $NR'_4$-Rest mit R' gleich Wasserstoff und/oder Alkyl- oder Arylrest und R ein gegebenenfalls substituierter Arylrest mit 6 bis 14 Kohlenstoffatomen sein kann, dadurch gekennzeichnet, daß man die entsprechenden Cyanoalkylphosphine zunächst mit einer wäßrig-alkoholischen Alkalimetallhydroxid-Lösung verseift, dann die wäßrige Lösung der Säuresalze mit einem sauren Ionenaustauscher behandelt und die freien Säuren gegebenenfalls mit einem Alkalimetallhydroxid oder Ammoniak bzw. einem Amin neutralisiert.

2. Verwendung der tertiären 2-Carboxiethyl- und Carboximethylphosphine nach Anspruch 1 als Katalysatorbestandteil bei der Oligomerisierung von Ethylen mit Hilfe eines Nickelkatalysators.